# EUROPEAN PATENT SPECIFICATION

(11) **EP 1 825 263 B1**
(45) Date of publication and mention of the grant of the patent: **22.10.2014**
(21) Application number: 05852321.8
(22) Date of filing: 30.11.2005
(51) Int. Cl.: G01N 33/53, C12M 1/00, C12N 5/00, C12N 5/02, C07H 21/02

(54) **CELLULAR SIGNALING PATHWAY BASED ASSAYS, REAGENTS AND KITS**
ZELLSIGNALISIERENDE TESTS, REAGENZIEN UND SÄTZE AUF GRUNDLAGE DES STOFFWECHSELWEGES
DOSAGES, REACTIFS ET KITS BASES SUR DES VOIES DE SIGNALISATION CELLULAIRE

(30) Priority: 30.11.2004 US 631435 P
(43) Date of publication of application: 29.08.2007
(73) Proprietor: Cell Networx LLC, Cambridge, MA 02139 (US)
(72) Inventor: PALMER, Michelle, Harvard, MA 01451-1834 (US)
(74) Representative: Sexton, Jane Helen
(86) International application number: PCT/US2005/042980
(87) International publication number: WO 2006/060335

(56) References cited:
- WO-A-01/81920
- WO-A-2004/094609
- WO-A-2005/023987
- US-A1- 2004 063 088
- SUCHTING STEVEN ET AL: "Soluble Robo4 receptor inhibits in vivo angiogenesis and endothelial cell migration" FASEB JOURNAL, vol. 18, no. 13, October 2004 (2004-10), XP009104203 ISSN: 0892-6638
- GUGGINO S ET AL: "Anti-angiogenic effect of aldosterone antagonist diuretics" ARCHIVES DES MALADIES DU COEUR ET DES VAISSEAUX 200207 FR, vol. 95, no. 7-8, July 2002 (2002-07), pages 727-731, XP009104204 ISSN: 0003-9683
- GOLDSTEIN ET AL: "Normal human fibroblasts enable melanoma cells to induce angiogenesis in type I collagen" SURGERY, C.V. MOSBY CO., ST. LOUIS, US, vol. 138, no. 3, 1 September 2005 (2005-09-01), pages 439-449, XP005101197 ISSN: 0039-6060
- YAN Y-X ET AL: "Cell-based high-throughput screening assay system for monitoring G protein-coupled receptor activation using beta-galactosidase enzyme complementation technology" JOURNAL OF BIOMOLECULAR SCREENING, LARCHMONT, NY, US, vol. 7, no. 5, 1 October 2002 (2002-10-01), pages 451-459, XP002278698 ISSN: 1087-0571
- GRAHAM D.L. ET AL.: 'Application of beta-galactosidase enzyme complementation technology as a high throughput screening format for antagonists of the epidermal growth factor receptor' JOURNAL OF BIOMOLECULAR SCREENING vol. 6, 2001, pages 401 - 411, XP008067451
- DE VOS F.Y.F.L. ET AL.: 'Endothelial cel effects of cytotoxics: balance between desired and unwanted effects' CANCER TREATMENT REVIEWS vol. 30, October 2004, pages 495 - 513, XP004621330
- ADES E.W. ET AL.: 'HMEC-1: establishment of an immortalized human microvascular endothelial cell line' JOURNAL OF INVESTIGATIVE DERMATOLOGY vol. 99, 1992, pages 683 - 690, XP000885610

## Description

### CROSS REFERENCE TO RELATED CASES

This application claims the benefit of Provisional U.S. Application Serial No. 60/631,435, filed November 30, 2004.

### BACKGROUND

### Technical Field

This application relates generally to an assay system to determine a drugs effect on a particular disease pathway, and methods of using this assay system.

### Background of the Technology

The much anticipated promise that, with a comprehensive blueprint of the human genome, many novel disease-specific molecular targets would be rapidly identified and that these would then form the basis of many new drug discovery programs has been slow to materialize. The greater number of genes and predicted gene products has created new challenges in the areas of target validation and lead optimization. As information about new genes grows, so does our understanding of the complexity of the signaling pathways and networks that govern the cellular biology. A comprehensive understanding of a gene's function in a cellular pathway will take years to unfold. Drug discovery research focused on a single isolated target runs the risk of identifying drugs that, when placed back into a cellular context, will show poor efficacy or adverse effects. Knowles, J., and G. Gromo, Nature Rev. Drug Discovery 2:63-69 (2003); Walters, W.P. and M. Namchuk, Nature Rev. Drug Discovery 2:259-266 (2003).

Complexity in pathways arises from the large number of components, redundancy in component functions, interactions among components, and temporal and spatial relationships between components. Pharmaceutical companies have recognized that although there are many potential targets, only a limited number will be viable small molecule targets and that they know very little about the role of those genes in the complex disease pathway. Hopkins, A.L. and C.R. Groom, Nature Rev. Drug Discovery 1:727-730 (2002); Drews, J., Science 287:1960-1964 (2000). Not only does the target need to be available to inhibition or activation by a highly selective small molecule entity, but it must also act at a node in the pathway that mediates the cellular response to the disease phenotype but not cause unwanted effects on related or networked pathways. These properties cannot be properly assessed in isolation outside the context of the cell and the critical nodes in those pathways must be measured in a manner that assesses their temporal relationship to one another.

A novel solution to the discovery of drugs is needed to meet the challenge of addressing the complexity and redundancy in the pathways that regulate the relevant disease biology. The multiple over-expressed, mutated, modified and translocated targets identified in patients leads to a change in how drug molecules should be discovered and optimized. A drug discovery tool that measures the targets as multiple nodes in the context of the dynamic cellular pathway would bridge the gap from *in vivo to in vitro* and bring the process of discovery closer to the reality of multi-targets identified in the patients. As an example, the success of Gleevec in cancer is not due to selective inhibition of ABL-BCR, but rather to the inhibition of multiple kinase targets in a manner that had not been predicted by standard discovery models. Indeed, it was discovered much later in development that Gleevec' s efficacy was due to it's inherent specificity for not only ABL-BCR but for c-Kit and PDGFr. O'Brien, S.G. et al, N. Engl. J. Med. 348:994-1004 (2003); Buchdunger, E. et al, J. Pharmacol. Exp. Ther. 295:139-145 (2000); Heinrich, M.C. et al, J. Clin. Oncol. 21:4342-4349 (2003). The multi-target challenge is not addressed by traditional drug discovery approaches. The pathway assay matrix (PAM) will provide a means by which to measure a drug effect in the context of the biological complexity. Integration of individual measurements of pathway node activity at relevant time points will provide a temporal fingerprint of pathway response. Pathway fingerprints will then be correlated with cellular responses, such as proliferation, migration, tubulogenesis and prostanoid synthesis.

### SUMMARY

The present invention provides a method of screening to identify inhibitors of angiogenesis comprising:
- treating primary cells or cell lines in tissue culture plate wells or vessels with a drug of interest selected from a small molecule, a siRNA, a protein, a peptide, an antibody, an antibody fragment, or an aptamer, wherein at least two different primary cells or cell lines are treated, each in their own tissue culture plate well or vessel;
- lysing the primary cells or cell lines;
- adding the cell lysates to eight or more wells of a microarray plate, with the caveat that cell lysate from only one primary cell or cell line is present in any single well;
- adding eight or more reagents to the wells of said microarray plate occupied by a primary cell, cell line or cell lysate, wherein at least one reagent is added to each of the wells of said microarray plate occupied by a cell lysate;
- conducting an assay reaction for each well of said microarray plate, wherein each reagent and or assay is chosen to measure a particular node in the disease pathway for the disease for which drugs are being screened, wherein at least eight assays are performed, and wherein said assays are 1) a KDR/VRAP or KDR/Gaq interaction cell based biosensor assay; 2) a FPRL- 1/β2-arrestin interaction cell based biosensor assay; 3) a src kinase assay; 4) p38 MAPK assay; 5) Ca2+ flux or cAMP production assay; 6) an Akt assay; 7) an ERK1 /2 cascade assay; and 8) an assay of phenotype (measure cell proliferation/cell survival); and
- analysing whether the drug of interest inhibits each of the nodes of the disease pathway, thereby determining whether the drug of interest would be effective as an inhibitor of angiogenesis.

### BRIEF DESCRIPTION OF THE DRAWINGS

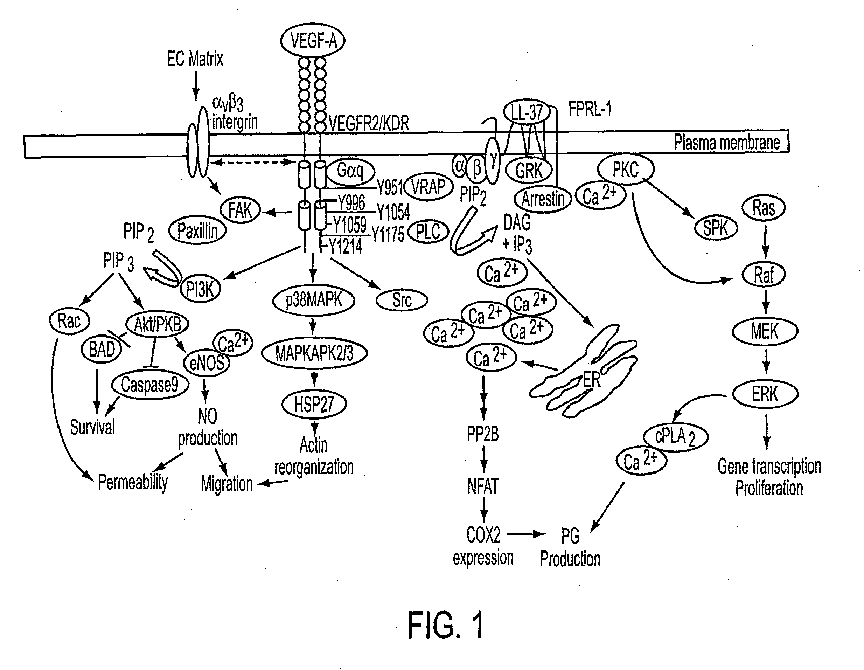

FIG. 1 Pathway map of angiogenic signaling in endothelial cells. The pathway map was adapted from the signaling map schematics in Zachery, I. (Biochem. Soc. Trans. 31 :1171-1177 (2003)) and Cross, MJ., et al. (Trends in Biochem. Sci. 28:488-494 (2003)). Abbreviations: cPLA2, cytosolic phospholipase A2; eNOS, endothelial nitric oxide synthase; Erk, extracellular regulated kinase; HSP27, heatshock protein 27; MAPKAP 2/3, MAPK-activating protein kinase-2 and 3; NO, nitric oxide; PGI2, prostacyclin; PIP3, phosphatidylinositol (3,4,5)-trisphosphate; Sck, Shc-like protein; SPK, sphingosine kinase; VEGF, vascular endothelial growth factor; VEGFR-2, vascular endothelial growth-factor receptor; VRAP, VEGFR-associated protein; PIP2, phosphatidylinositol (4,5)-bisphosphate; DAG, sn-1,2-diacylglycerol; IP3, inositol (1,4,5)-trisphosphate; PKC, protein kinase C; ER, endoplasmic reticulum; PI3K, phosphoinositide 3-kinase; FAK, focal adhesion kinase; p38MAPK, p38 mitogen-activated protein kinase; GRK, G protein coupled receptor kinase.

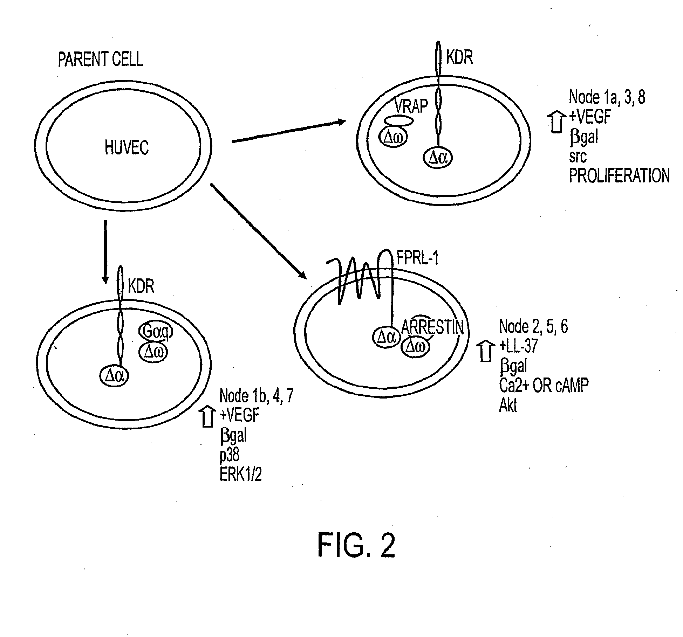

FIG. 2 Schematic of the assay development strategy. The parental cell expresses full length KDR fused to one biosensor component or FPRL-1 fused to one biosensor component. The interacting partner fused to the second biosensor will be infected into the receptor cell line. Two biosensor cell lines (Node 1a or Node 1b and Node 2) are planned but only two will be selected for incorporation into the matrix. Selection of biosensor cell lines nodes 1a and 1b will depend on ease of vector construction and expression of the fusion protein. The remaining 6 nodes can be measured in any of the three biosensor cell lines (KDR/VRAP or KDR/Gαq and FPRL-1/β2-arrestin) and will measure endogenous levels of the node target proteins.

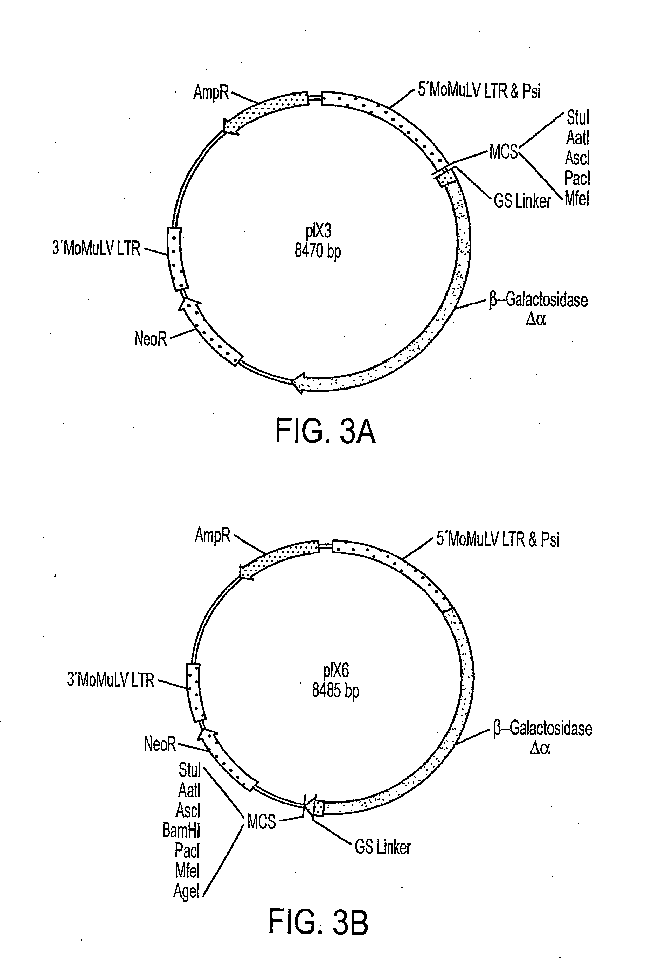

FIGS. 3A-3D The InteraX™ system vectors. pIX3/6 and pIX9/12 vectors are retroviral vectors and contain the 5' and 3' long terminal repeats (LTRs) and viral packaging signal (Psi+) from the Moloney Murine Leukemia Virus (MuMoLV) for stable insertion of DNA sequence into a mammalian genome. The LTRs also contain sequences for promoter and processing functions. The pIX3/6 vectors contains the *AmpR* (Ampicillin resistance) gene for selection in *E. coli* and contains the neomycin resistance gene for selection of stable mammalian cell lines in the presence of Geneticin™ (G418) antibiotic. The pIX9/12 vectors contain the *AmpR* gene for selection in *E. coli* and contains the hygromycin resistance gene for selection of stable mammalian cell lines in the presence of hygromycin.

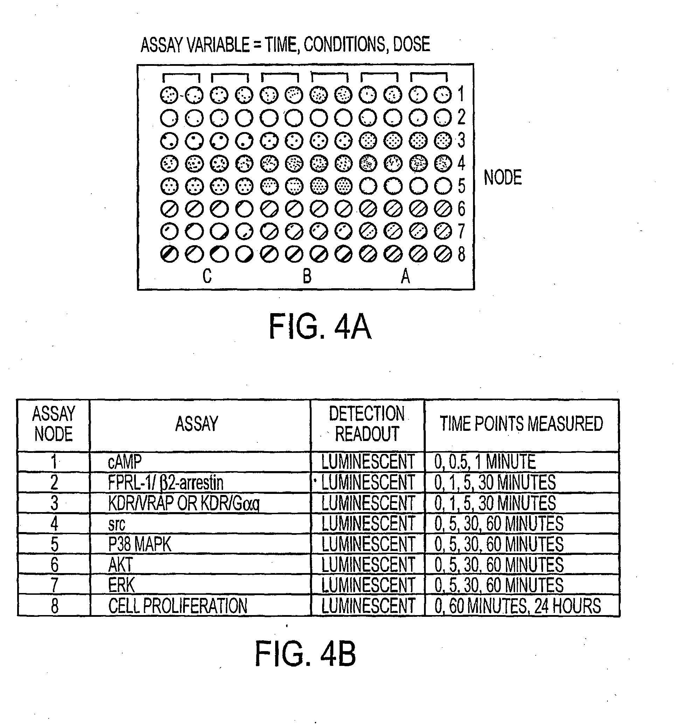

FIGS. 4A and 4B A depiction of a possible arrangement of a 96 well plate used in the invention. Nodes are grouped by: pathway time scale (early or late events), assay detection mode (fluorescence, chemiluminescence), assay workflow (number of additions, detection in plate or off plate). A,B,C = time point, assay conditions (+/ligand), compound, dose of compound, RNAi.

### DETAILED DESCRIPTION

Disclosed herein is a novel assay system to identify drugs for the treatment of diseases. This novel approach involves probing a multi-target disease pathway, rather than a single target. By using a more physiologically relevant assay system for drug screening, one will yield a more efficacious drug candidate, reduce costs and shorten timelines compared to conventional drug discovery approaches.

Complexity in pathways arises from the large number of components, redundancy in component functions, interactions among components, temporal and spatial relationships between components. Pharmaceutical companies have recognized that although there are many potential targets, only a limited number will be viable small molecule targets and that they know very little about the role of those genes in the complex disease pathway. (Hopkins, A.L. and C.R. Groom, Nature Rev. Drug Discovery, 1:727-730 (2002); Drews. J., Science, 287:1960-1964 (2000).) Not only does the target need to be available to inhibition or activation by a highly selective small molecule entity but it must also act at a node in the pathway that mediates the cellular response to the disease phenotype but not cause unwanted effects on related-or networked pathways. These properties cannot be properly assessed in isolation outside the context of the cell and the critical nodes in those pathways must be measured in a manner that assesses their temporal relationship to one another.

The assay system is called the pathway assay matrix (PAM). PAM will provide a means in which to measure a drugs effect in the context of the biological complexity. The PAM system is built on a combination of pathway nodes that are implicated in disease based on data showing over expression, mutation, modification or delocalization, and other nodes are chosen for their importance in monitoring the signaling strength and pathway dynamics in the cellular model. This unbiased approach allows for the selection and optimization of multi-targeted compounds. PAM utilizes an optimized combination or matrix of assay technologies in a high throughput microplate format to monitor multiple points or nodes in the disease pathway simultaneously. The approach does not define a single target up front; rather, it lets the compound's effect on the pathway determine the best target or combination of targets. The effect of an inhibitor or stimulant on a pathway would result in a unique fingerprinting derived from pathway node data. Pathway fingerprints will then be correlated with cellular responses such as proliferation, migration, tubulogenesis and prostanoid synthesis. The pathway node fingerprint in cell and animal models will be used to guide optimization of the drug lead. Fingerprint analysis has been successfully applied in genomic and proteomic analyses to identify novel biomarkers. Baily, W.J. and R. Ulrich, Expert Opin. Drug Saf., 3:137-151 (2004); Livesey, F.J. et al., Proc. Natl. Acad. Sci. U.S.A., 101:1374-1379 (2004); Gerritsen, M.E. et al., Microcirculation, 10:63-81 (2003).

PAM merges target selection and validation with lead identification, thus casting a wider target net and at the same time reducing the time required for novel drug discovery. PAM is also likely to yield data that better predicts performance in animal *models in* vivo. The culmination of steps in a signaling pathway results in a specific outcome or phenotype, such as protein protein interaction, protein phosphorylation, cell proliferation and migration. Correlation of assay data with phenotype is a step closer to understanding the *in vivo* animal model results. Better predictive assay tools at an earlier point in the discovery process will improve drug candidate selection, and provide better biomarkers for *in vivo* testing.

The PAM system for screening drugs to determine whether they are effective for treating or preventing a disorder comprises a microarray plate. Although the standard size microarray plate has 96 wells, other sizes of microarray plates can be used, such as a 384 and 1536 well plates. Microarray plates include plates that are intended for tissue or cell culture, i.e. tissue culture plates. Hereinafter, when referring to the lanes of a microarray plate, the number of lanes will depend on the orientation of the plate. For example, with a 96 well plate, there are either 8 lanes of 12 rows each, or 12 lanes or 8 rows each, depending on the orientation. If, for example, the pathway being studied by PAM has more than 8 nodes, it may be beneficial to orient the plates so that there are 12 lanes of 8 rows. The reagents for each node assay may be used in replicates of 2 or more.

The PAM system also comprises a treated primary cell, cell line or cell lysate in each well of the microarray plate being used for PAM, with the caveat that there is more than one treated primary cell, cell line or cell lysate distributed in the wells of the microarray plates, and only one treated primary cell, cell line or cell lysate is present in any single well. Preferably, at least six wells of the microarray plate are used. By cell line it is meant a defined population of cells which has been maintained in a culture for an extended period and which has usually undergone a spontaneous process of transformation conferring an unlimited culture lifespan on the cells. Primary cells are cultured cells that are derived directly from tissue (often embryonic tissue). They are distinct from transformed cell lines. Both cell lines and primary cells can be modified recombinantly by the expression of a target gene.

By "treated primary cell, cell line or cell lysate" it is meant that the primary cell or cell line or cell lysate has been exposed to a condition in a cell culture before adding to the wells or the microarray plates and performing the node assays. A treated cell lysate would be a cell lysate obtained by lysing the primary cells or cell lines exposed to a drug or compound of interest in a cell culture. By "condition" it is meant a drug or compound of interest, ligand, RNAi, growth conditions (media, temperature, pH, O₂). By "drug or compound of interest" it is meant one or more drugs or compounds of interest. The amount of drug or compound used will be dependent upon the screening conditions being used, and thus may vary for each treated cell. For example, many compound libraries are prepared at 1 mg/ml, but concentration would vary with molecular weight of a given compound.

The PAM system may also comprise an untreated primary cell, cell line or cell lysate in one or more wells of the microarray plate. The untreated primary cells, cell lines or cell lysates would act as a control for the assays being performed. In other words, for each node of the disease pathway being tested, there should be at least one untreated primary cell, cell line or cell lysate used as a control, and the assay being performed to test the activity of the drug of interest on that particular node of the disease pathway should also be performed on the control.

Although, for example, 96 wells may be present in the plate, it may not be necessary to use all 96 wells. One node may take up more than one lane. Furthermore, every well within a lane may not be used if there are fewer assays to be performed than wells available in the lane. Thus, a primary cell, cell line or cell lysate would only be present in a well of the microarray plate which will be used for a particular assay. The amount of primary cell, cell line or cell lysate present in the well may vary according to what assay is to be performed in that particular well. One of skill in the art would know how much of a primary cell, cell line or cell lysate needs to be used for each of the assays performed in PAM.

Although only one treated primary cell, cell line or cell lysate will be present in any one well of the microarray plate, there must be two or more treated primary cells, cell lines or cell lysates used in the PAM system. The condition tested may be drug concentration, ligand concentration, presence of one or more RNAi molecule, growth conditions, treatment time points. For example, for the first lane, cell line treated with condition A is present. For the second lane, cell line treated with condition B is present. For the third lane, cell lysate treated with condition C is present, *etc.* In other words, multiple treated primary cells, cell lines or cell lysates may be present in the wells of the microarray plate, in any configuration, so long as each individual well only has one treated primary cell, cell line or cell lysate. Although the arrangement of the lanes in the microarray plate is left up to the individual scientist performing PAM, it is suggested that the lanes be organized according to what type of assays are being performed. Thus, if two nodes of the pathway require PCR analysis, then those lanes of the microarray plate should be located next to it. Alternatively, one may organize the microarray plate chronologically according to the appearance of the node in the pathway. Thus, node one of the pathway would be lane one, node two of the pathway would be lane two, etc. Of course, if a single node in a pathway requires more than one lane, then the organization of the microarray plate can be adjusted accordingly.

The PAM system also comprises a reagent in each of the wells of the microarray plate occupied by a treated primary cell, cell line or cell lysate. More than one reagent may be present in each well (thus, by "a reagent" it is meant one or more reagents). Preferably, six or more reagents are used in the PAM system. The reagent(s) used will be dependent upon the assay being performed in that particular well. Thus, if in lane one, row one, a kinase assay is performed, kinase assay reagents (anti- phospho antibody, capture antibody, secondary antibody, chemiluminescent detection reagent) would be present in the well. If in lane two, row one, reporter gene assay is to be performed, reporter gene reagents (beta-galactosidase substrate, *etc*.) would be present in the well, *etc.*

The PAM system may further comprise a ligand. By "a ligand" it is meant one or more ligands. The amount of ligand used will be dependent upon the node assays used, and thus may vary the condition. Ligands that can be used in the PAM system include, but are not limited to, growth factors, cytokines, chemokines, peptides, hormones, lipids, receptors, soluble receptors, small molecules, antibodies, antibody fragments, aptamers, nucleic acids, polymers, carbohydrates, amino acids.

"Drugs" or "compounds" to be analyzed in the methods of the present invention include, but are not limited to, small molecule compounds, siRNAs, proteins, peptide, antibodies, antibody fragments and aptamers.

Cell lines to be used in the assay systems and methods of the present invention include, but are not limited to, cell lines 1321N1; 143B; 23132/87; 293; 2A8; 32D; 3A9; 3T3; 4T1; 5838Ewing's; 661W; 697; 721.174; 721.22; 721.221; A-10; A-431; A172; A2.A2; A20; A2780; A3.01; A375; A549; A7r5; AGS; AML; ARH77; ARPE19; ASZ001; AT-1; ATDC5; AtT20; B-CLL; b-END; B65; BA/F3; BC-3; BCBL1; BCL1.3B3; BEAS-2B; BHK-21; BHP2-7; BJ; BJ1-hTERT; BJAB; BJMC3879; BL2; BL3; BLCL; BPH1; BT-20; BT549; BV173; BV2; BW5147; C10/MJ2; C17.2; C28A2; C2C12; C2F3; C57MG; C6; CaCo-2; Calu-3; CCRF-CEM; CEM.C1; CH1; CH12; CH27; CHO[suspension]; CHO-K1; Colo201; Colo205; Colo357; COS-1; COS-7; CV1; D54; DEV; DHL4; DHL6; DOHH-2; DOV 13; DT40; DU 145; EAhy926; cCAS; EcR293; ECV304; EL4; EpH4; F36P; F9; FaO; FDC-P1; FL5.12A; GD25; GH3; GIST882; GM05849; GT1-7; H4; H4IIE; H69; HaCaT; HCA7; HCT116; HCT15; HDLM-2; HEL; HeLa; Hep-2; Hep1B; HEPA1-6; Hepatocyteimmortalized,mouse; HepG2; HFF,immort.; HFFF2; HIB1B; HK2; HL-1; HL-60; HMEC-1; HN5; HPB-ALL; HT-1080; HT-29; HT22; HT29-D4; HTC; HU609; HuH7; HuT78; HuT102; IEC 18; IGROV1; IHH; IM9; IMR-32; IMR-90; TNS1; INS1832/13; IOSE29; IOSE80; J-774; J558L; J774A.1; J774e; JB6-1; JB6-2; Jurkat; K562; Karpas299; KG-1; KG-1a; KM-H2; KS; KTA2; L1.2; L1210; L1236; L428; L540; L929; LAMA-84; LAZ221; LCL; LLC-MK2; LLC-PK10; LNCaP; LP 1; LS 180; LX-2; M-07e; M28; MA104; MC3; MC3T3-E1; MC57G; MCF-7; MCF-7tet; MCF10; MCF10A; MCT; MDA-MB-231; MDA-MB-361; MDA-MB-453; MDA-MB-468; MDBK; MDCK; MDCKII; MDCK-C7; ME-1; MedB1; MEG01; MEL; melan-a; Meso17; MEWO; MFM223; MGR3; MHP36; MiaPaCa; mIMCD3; MIN6; Mino; MLO-Y4; MM.1S; MOLT-4; Molt16; MonoMac1(MM1); MonoMac6(MM6); MouseLcell; MPC 11; MPRO; MRC-5; MT4; MUTZ3; MzCHA-1; N114P2; N1E115; NALM-6; Namalwa; NB-4; NBL-6; NCEB; NCI-H1299[H1299]; NCI-H266[H266]; NCI-H295R[H295R]; NCI-H358[H-358;H358]; NCI-H460[H460]; NCI-H69[H69]; NCI-H929[H929]; NCM460; Neuro-2a(N2a); Neuroblastoma; NIH/3T3; NIH/3T3; NK3.3; NKL; NKL1; NRK; NRK52E; NSO; NS1; NSC34; OCI-AML1a; OCI-AML2; OVCAR3; ; P19; P3X63Ag8; P815; PANC-1; Panc89; PC-12; PC-3; PLB-985; PMC42; PS1; PtK1; R28; RAEL; Raji; Ramos; Rat2; RAW264.7; RBL; RBL2H3; RCC26; REH; RFL-6; Rh4; RKO; RMAS; RPMI8226; RS4-11; RT4; S1A.TB.4.8.2; S2; S49; SA1N; SAOS-2; SbCl2; Schwannomacellline; SCID.adh; SH-SY5Y; SK-MEL-28; SK-N-SH; SK-OV-3; SKBR3; SKNAS; SKNMC; SKW6.4; SP53; SUP-T1; SW13; SW1353; SW48; SW480; SW872; SZ95; T-47D; T0; T1165; TF1; TG40; THP-1; TOM-1; Tot2; TS/A; U-2OS; U-87MG; U138MG; U251MG; U266B1; U937; UACC903; UMSCC-14A; UT7; UT7GM-CSFdependent; UT7-Epo; UT7-EpoS1; UT7-TPO; V79; Vero; WEHI-231; WM35; XG6; YT; YTS) and primary cells (Adipocyte(pre),human; Astrocyte,mouse; Astrocyte,rat; BCell,human; Cardiomyocyte,rat; CD34+Cell,human; Chondrocyte,human; Dendritic cell(DC),human; Embry.fibroblast(MEF),mouse; Embry.stem(ES)cell,mouse; Endo.,cor.art.(HCAEC),human; Endo.,lung(HMVEC-L),human; Endo.,neo.derm.(HMVEC-d),hum; Endo.,umbil.vn.(HUVEC),human; Endo.,umbil.vn.(HUVEC),human; Endothelial,aortic,human; Epith.,bronch.(NHBE),human; Epith.,bronch.(NHBE),human; Epith.,bronchial,monkey; Epith.,mammary(HMEC),human; Epith.,mammary(HMEC),human; Epith.,prost.(PrEC),human; Epithelial,airway,human; Epithelial,alveolar,rat; Epithelial,cornea,human; Epithelial,kidney,human; Epithelial,mammary,mouse; Fibr.,derm.(NHDF-adult),hum; Fibr.,derm.(NHDF-neo),human; Fibroblastforeskin,human; Fibroblast tunica albug,human; Hepatocyte,mouse; Hepatocyte,rat; Keratinocyte,adult human; Keratinocyte,neonatal,human; Macrophage,human; Macrophage,mouse-BALB/c; Macrophage,mouse-C57BL/6; Melanocyte,(NHEM-neo),human; Mesench.stem(MSC),human; Monocyte,human; Myofibroblast,human hepatic; Myofibroblast,rathepatic; Naturalkiller(NK),human; Neural stem cell(NSC),mouse; Neural stem cell(NSC),rat; Neuron,mouse; Neuron,rat; Oligodendrocyte,rat; Podocyte; Renal prox.tubule cells,human; Sm.mus.,aortic(AoSMC),human; Sm.mus.,aortic(AoSMC),rat; SMC,coronary artery,human; Smooth muscle(SMC),rat; Stromalcells,humancervical; TCell,mouse-BALB/c; TCell,mouse C57BL/6; TCell,stim.,human; TCell,unstim.,human; Trophoblast,mouse), recombinant modification of any of these cells or any eukaryotic cell line that is useful for studying a disease pathway. Preferably, the cell line would be a human cell line when a human disease pathway is being tested.

Reagents to be used in the assay systems and methods of the present invention include, but are not limited to, essential and nonessential amino acids, inorganic salts, organic compounds, trace elements, radionucleotides, serum, growth factors, antibiotics, vitamins, oligonucleotides, and polymerases.

Also disclosed herein are methods of screening for compounds for their effectiveness for treating or preventing a disease comprising treating one, two or more primary cells or cell lines in wells or vessels of a tissue culture plate with a condition (as defined above) and optionally lysing the treated primary cells or cell lines, and adding the treated primary cells, cell lines or cell lysates to two, three, four, five, six, seven, eight, nine, ten, eleven, twelve, thirteen, fourteen, fifteen, sixteen, seventeen, eighteen, nineteen, twenty, twenty-one, twenty-two, twenty-three, twenty-four, twenty-five, twenty-six, twenty-seven, twenty-eight, twenty-nine or thirty wells of a microarray plate, preferably 6 or more wells of a microarray plate, with the caveat that only one primary cell, cell line or cell lysate is present in any single well. The wells of the microarray plate may contain six or more reagents (at least one reagent in each of the wells of the microarray plate to which primary cells, cell lines or cell lysates are to be added). Alternatively, the reagents are added to the wells after the primary cells, cell lines or cell lysates. In yet another alternative embodiment, the reagent(s) are added to the tissue culture plate with the treated primary cells, cell lines or cell lysates prior to transferring the primary cells, cell lines or cell lysates to the microarray plates. Once the reagents and the primary cells, cell lines or cell lysates have been added to the microarray plate, an assay is conducted and its outcome measured (by fluorescence, colormetric, chemiluminescent, label free, radiometric readout, etc.) At least two, three, four, five, six, seven, eight, nine, ten, eleven, twelve, thirteen, fourteen, fifteen, sixteen, seventeen, eighteen, nineteen, twenty, twenty-one, twenty-two, twenty-three, twenty-four, twenty-five, twenty-six, twenty-seven, twenty-eight, twenty-nine or thirty assays are performed, representing two, three, four, five, six, seven, eight, nine, ten, eleven, twelve, thirteen, fourteen, fifteen, sixteen, seventeen, eighteen, nineteen, twenty, twenty-one, twenty-two, twenty-three, twenty-four, twenty-five, twenty-six, twenty-seven, twenty-eight, twenty-nine or thirty nodes of a pathway. Preferably, at least six assays are performed, representing at least six nodes of a pathway. Each reagent and/or assay used in PAM is chosen to measure a particular node in the disease pathway for the disease for which drugs are being screened Once the assay reactions are performed, one of skill in the art would analyze the data to see if a drug or compound inhibits or effects each of the six or more nodes tested in the PAM disease pathway, generating a fingerprint of node activity. The drug fingerprint would indicate how effective a drug was at modifying a disease pathway by inhibiting one or a combination of targets and therefore may be useful in methods of treating or preventing that disease. The drug fingerprint will also provide data to improve specificity and potency of a drug through medicinal chemistry approaches. As discussed above, the primary cells, cell lines, cell lysates and reagents used in this method would change depending on what type of assay is going to be performed in a particular well. Ligands (as defined above) may also be added to the one or more wells of the microarray plates.

In an alternative embodiment, the treated primary cells, cell lines or cell lysates are separated into six or more well or vessels of the tissue culture plate, and the reagents, ligands, etc. are added thereto. The assays would then be performed on the treated primary cells, cell lines or cell lysates in the wells or vessels of the tissue culture plate. In this embodiment, there are optionally untreated primary cells, cell lines or cell lysates in six or more wells or vessels of the tissue culture plate, and each of the assays performed on the treated primary cells, cell lines or cell lysates are also performed on the untreated primary cells, cell lines or cell lysates as a control.

The assay technologies used in PAM include, but are not limited to, protein protein interaction assays (including BRET, FRET, PCA, InteraX, immunoprecipitation assays, NMR, mass spectrometry, two-hybrid assay, SPR, BIND, ELISA, ELISPOT, bead based capture methods (i.e. Luminex), FMAT, SPA, scintillation microplates, reporter gene assay, non-denaturing gel shift assay); protein DNA or RNA interaction assays (including BRET, FRET, PCA, InteraX, immunoprecipitation assay, NMR, mass spectrometry, two-hybrid assay, SPR, BIND, ELISA, ELISPOT, bead based capture methods (i.e. Luminex), FMAT, SPA, scintillation microplates, reporter gene assay, gel shift assay, capillary electrophoresis); protein small molecule interaction assays (including immunoprecipitation, NMR, mass spectrometry, two-hybrid assay, SPR, reporter gene assay; BIND, ELISA, ELISPOT, bead based capture methods (i.e. Luminex), FMAT, SPA, scintillation microplates, capillary electrophoresis); kinase assays (including microfluidics, microarray, ELISA, SPA, scintillation plates, radiometric arrays, HPLC, ATP depletion assay, kinase cascade assay, FRET, HTRF TRF, DiscoverX, PCA, InteraX, SPR, reporter gene assay, biosensor assays, western blot); protease assays (including FRET, fluorescent assays, HPLC, microfluidics, ELISA, radiometric, SPA, scintillation plates, western blot); phosphatase assays (microfluidics, microarray based assay, ELISA, SPA, scintillation plates, radiometric, HPLC, FRET, fluorescent, HPLC, microfluidics, ELISA, radiometric assays); glycolytic enzyme assays (including fluorescent assays, chemiluminescent assays, HPLC, microfluidics, ELISA, radiometric assays, SPA, scintillation plates, other radiometric assays); reductase assays (including fluorescent assays, chemiluminescent assays, HPLC, microfluidics, ELISA, radiometric assays, SPA, scintillation plates, other radiometric assays); phospholipase assays (including fluorescent assays, chemiluminescent assays, HPLC, microfluidics, ELISA, radiometric assays, SPA, scintillation plates, other radiometric, assays); oxidase assays (including fluorescent assays, chemiluminescent assays, HPLC, microfluidics, ELISA, radiometric assays, SPA, scintillation plates, other radiometric assays); esterase assays (including fluorescent assays, chemiluminescent assays, HPLC, microfluidics, ELISA, radiometric assays, SPA, scintillation plates, other radiometric assays); ligase assays (including fluorescent assays, chemiluminescent assays, HPLC, microfluidics, ELISA, radiometric assays, SPA, scintillation plates, other radiometric assays); dehydrogenase assays (including fluorescent assays, chemiluminescent assays, HPLC, microfluidics, ELISA, radiometric assays, SPA, scintillation plates, other radiometric assays); enolase assays (including fluorescent assays, chemiluminescent assays, HPLC, microfluidics, ELISA, radiometric assays, SPA, scintillation plates, other radiometric assays); nuclease assays (including fluorescent assays, chemiluminescent assays, HPLC, microfluidics, ELISA, radiometric assays, SPA, scintillation plates, other radiometric assays); deaminase assays (including fluorescent assays, chemiluminescent assays, HPLC, microfluidics, ELISA, radiometric assays, SPA, scintillation plates, other radiometric assays); polymerase assays (including fluorescent assays, chemiluminescent assays, HPLC, microfluidics, ELISA, radiometric assays, SPA, scintillation plates, other radiometric assays); transferase assays (including fluorescent assays, chemiluminescent assays, HPLC, microfluidics, ELISA, radiometric assays, SPA, scintillation plates, other radiometric assays); topoisomerase assays (including fluorescent assays, HPLC, microfluidics, ELISA, radiometric assays, SPA, scintillation plates, other radiometric assays); protein modification assays (including phosphorylation, lipidation, proteolytic cleavage, capping, and glycosylation assays); metabolite assays (including fatty acid uptake assays, and glycolysis assays); gene expression assays (including PCR, TaqMan, bDNA, Hybrid capture, FISH and reporter gene assays); protein expression assays (ELISA, microarray assays, mass spectrometry, SPR, BIND, reporter gene assays); apoptosis assays (measured by Annexin binding, caspase, Tunel, DNA fragment, membrane permeability, and mitochondrial membrane potential); toxicity assays (ATP, dBrd incorporation, and mitochondrial membrane potential); cell proliferation assays (ATP, dBrd incorporation, radiometric assays, telomere length, and mitochondrial membrane potential); translocation assays (using DiscoverX, InteraX, PCA, fluorescent microscopy, fluorescent cellular imaging, fluorescent proteins, or antibodies); cell motility assays (including conductance assays, membrane potential assays, and chamber assays); cell adhesion assays (including ELISA, flow cytometry, FMAT, reporter gene assays, and kinase assays); cell invasion assays (including membrane assays, collagen assays, and ECMatrix cell invasion assays); morphology assays (including microscopy assays and cell imaging assays); membrane potential assays (including fluorescent assays, chemiluminescent assays, microfluidics); and second messenger assays (including Ca, cAMP, IP3, cGMP, prostaglandins, leukotrienes, phospholipids, arachidonic acid, and NO assays). One of skill in the art would know how to perform each of these assays.

When the assay being performed is a western blot or PCR assay, or any similar assay, that would require the running of a gel or the performance of additional steps after the assay reaction has taken place, then the methods of the invention would further comprise performing such steps, such as running a gel, *etc.*

Although the examples provided are directed to the use of PAM for identifying drugs for the treatment of diabetic retinopathies and age related macular degeneration, PAM can be used to identify drugs for the treatment of other diseases, and to identify drugs that effect other pathways, as well. Signal transduction and regulatory pathways that would be used in the present invention include, but are not limited to, chromatin regulation and acetylation; MAPK signaling (Mitogen activated protein kinase (and mutant MAPK) signaling cascades stimulated by growth factors, G-protein coupled receptors, mitogens, growth factors, and inflammatory cytokines); apoptosis, cell death and receptor signaling of Fas/CD95, TNFR-1, TNFR-2, APO-3L, APO-2L, their effector molecules FADD, TRAF2, Daxx, TRADD, RIP, and their mutants; Caspase apoptosis, cell death and receptor signaling of survival factors such as growth factors and cytokines, and their mutants, or removal of survival factor; Akt/PKB (and their mutants) signaling pathways and downstream effectors stimulated by GPCR, RTK, integrin, and cytokine receptor activation; pathways regulating translational control stimulated by stress factors such as TNFa, IL-1b, heat shock, anisomycin, arsenite, oxidative stress, amino acid deficiency, hypoxia, UV damage, heme defficiency or receptor activation by ligands such as growth factors, hormones, cytokines, neuropeptides and effecting p38mapk, erk, pdk1/2, akt, GSK3b, PKR, PERK, GCN2, HRI, elF2, elF4, p70S6 kinase and downstream signaling molecules, including mutants; PKC/phospholipase (and their mutants) activation due to stimulation of inflammatory cytokine receptors, GPCRs, and growth factor receptors; stimuli such as growth factor withdrawal, UV stress response, replicative senescence, TGFb, contact inhibition, DNA damage that effects cell cycle/checkpoint and effecting activity of p53, myc, smad, ATM/ATR, HIPK2, DNA-PK, p16INK4a, p18 INK4c, p19 INK4d, p15 INK4b, p27 KIP1, p21 CIP1, Chk1/2, cdc25a, cdc25B/C, GSK-3b, CDK2, CDK4/6, cdc2, cyclin B, cyclin D, cyclin E, and all downstream effectors and mutants; Jak/Stat pathway, downstream effectors (and mutants) stimulated by IL6 receptor/gp130 activation and growth factor cross talk (*i.e.* EGFR); NF-kappaB signaling stimulated by activation of B cell receptor, T cell receptor, IL-1r, TNFr, growth factor receptors (*i.e.* BMP, EGF, HGH, insulin, NGF, TGFα), LTβr, CD40, BR3 and their downstream effector molecules, and mutants; TGF-β/SMAD signaling via receptor stimulation and downstream effectors including transcription factors, co-repressors and co-activators, and their mutants; cytoskeletal and cell-cell contact signaling effecting actin cytoskeleton reorganization, cell motility, survival, growth, microtubule polymerization and the pathway molecules involved in signaling; glucose metabolism or inhibition of sterol/isoprenoid synthesis, fatty acid oxidation via AMPK signaling stimulated by hypoxia, ischemia, heat shock, adiponectin, and leptin; insulin receptor signaling and cross talk with TNFr1, Glut4 and SNARE complex and effects on downstream signaling molecules (including mutants) effecting sodium transport, apoptosis, glycogen synthesis, fatty acid synthesis, lipolysis, and protein synthesis growth; lymphocyte signaling via B cell antigen receptor and T cell receptor signaling including downstream signaling molecules and cross talk with CD19, CD22, CD45, CD4, CD28, LFA-1, calcium channels and their downstream signaling molecules, and mutants; nuclear receptor activation via ligands, interactions with co-activators and co-repressors, and transcription/repression of gene expression; Wnt/β-catenin signaling via frizzled receptor including adaptor molecules and downstream signaling molecules, and their mutants; receptor Tyr kinases (KDR, KIT, FLT5 PDGFRa and b, Tie2, FGFR 1 -4, Ret, EphB, EGFR3 HER 2-4, IGF 1 -R, ALK5 INS-R ad their mutants) and cytoplasmic tyrosine kinase (jak 1-3, tyk, csk, lyn, src, fyn, fcr, blk, fak, pyk2, syk, zap-70, abl, btk and their mutants) signaling pathways, including adaptor proteins such as cbl, gab, crk, dok, grb, and she.

Also disclosed are kits for screening drugs to determine whether they are effective for treating or preventing a disease. These kits comprise a microarray plate, two or more cell lines or cell lysates, and at least one reagent.

### Background on Diabetic Retinopathies and Age-Related Macular Degeneration

As the population ages the number of people over 50 years of age with conditions leading to the loss of eyesight is increasing. Two main disease contributors to this loss of sight are proliferative diabetic retinopathies (DR) and age-related macular degeneration (AMD). Clark, A.F. and T. Yorio, Nature Rev. Drug Discovery, 2:448-459 (2003); Witmer, A.N. et al, Progress in Retinal and Eye Research, 22:1-29 (2003). Each year, 1.3 million Americans above the age of 20 are diagnosed with diabetic retinopathy which over time can progress to proliferative retinopathy. Progression of the disease is aided by circulation problems which cause areas of the retina to become oxygen-deprived or ischemic. The condition is characterized by the development of neovascularization or new, abnormal vessel growth. Another disease characterized by abnormal blood vessel formation in the eye is the wet form of AMD. According to the National Eye Institute (NEI), more than 1.7 million people in the United States have AMD. One in twenty senior citizens are presently affected by age-related macular degeneration, and as people live longer and the baby-boomers reach retirement, the frequency is expected to exceed 2.95 million individuals by 2020. The wet form of AMD is less common (10%), but it is typically more damaging, and leads to loss of vision.

The role of angiogenesis and VEGF-A, its key mediator, in ischemia-induced intraocular neovascularization has been directly demonstrated in various animal studies using various VEGF inhibitors. Witmer, A.N. et al.; Aiello, L.P. et al., Proc. Natl. Acad. Sci. U.S.A., 92:10457-10461 (1995); Adamis, A.P. et al., Arch. Ophthalmol., 114:66-71 (1996); Robinson, G.S. et al., FASEB J., 15:1215-1217 (2001); Ozaki, H. et al., Am. J. Pathol., 156:697-707 (2000); Campochiaro, P.A. and S.F. Hacckett, Oncogene, 22:6537-6548 (2003); Ferrara, N., Endocrine Reviews, 25:581-611 (2004). A number of anti-angiogenic molecules targeting VEGF for treatment of neovascular eye disorders are currently being explored in AMD patients, including a recombinant humanized anti-VEGF Fab (rhuFab VEGF) and 2-fluoropyrimidineRNA oligonucleotide ligand aptamer. Witmer, A.N. *et al.;* Chen, Y. et al., J. Mol. Biol. 293:865-881 (1999); Ruckman, J. et al., J. Biol. Chem., 273:20556-20567 (1998). Results of a clinical study with the aptamer, Macugen (Pegaptanib) in patients with wet AMD indicate reduced vision loss compared with placebo. However, the magnitude of the effect did not appear markedly greater than that achieved with photodynamic therapy. Ferrara, N., Endocrine Reviews, 25:581-611 (2004); Doggrell, S.A., Expert Opin. Pharmacother., 6:1421-1423 (2005). Lucentis, a humanized antibody fragment developed at Genentech and designed to bind and inhibit Vascular Endothelial Growth Factor A, is demonstrating improved vision in Phase III trials in patients with wet AMD. Michels, S. and P.J. Rosenfeld, Klin. Monatsbl. Augenheilkd., 222:480-484 (2005). These treatments require repeated delivery of the drug to the eye by intravitreous injection. Treatment would benefit from a small molecule therapeutic that could be delivered locally to the eye, but it would need to overcome specific blood-aqueous and blood-retina barriers. Clark, A.F. and T. Yorio, Nature Rev. Drug Discovery, 2:448-459 (2003). Other anti-angiogenic targets and molecules in development are currently limited to VEGF-A inhibitors, PKC-beta inhibitors, integrin antagonists and steroid therapy. Witmer, A.N.; Ferrara, N. Small molecule inhibitors of PKC beta and VEGFR2/KDR are being evaluated in clinical trials. Compochiaro, P.A., Invest. Opthalmol. Vis. Sci., 45:922-931 (2004). Recent findings in clinical studies have shown that a number of the anti-angiogenic agents developed to date have yielded disappointing results in clinical studies. Ton, N.C. and G.C. Jayson, Curr. Pharm. Des., 10:51-64 (2004); Shepherd, F.A. and S.S. Sridhar, Lung Cancer, 41:63-72 (2003).

The discrepancy between the experimental and clinical finding is being attributed to heterogeneity and redundancy in the VEGF signaling pathway. Multiple receptors and growth factors have been implicated in mediating angiogenesis (for a list of these factors see Witmer, A.N. et al., Progress in Retinal and Eye Research, 22:1-29 (2003); and Ferrara, N. Endocrine Rev., 25:581-611 (2004).) Not only is the VEGFR2/KDR-mediated cascade complicated (Figure 2) but other VEGF receptors, VEGFR1/Flt-1 and Neuropilin-1 (NP-1) further complicate the picture. (Zachery, I., Biochem. Soc. Trans., 31:1171-1177 (2003); Cross, M.J., et al., Tr. Biochem. Sci., 28:488-494 (2003).) These interactions are not yet understood in detail. Control of angiogenesis is regulated by a dynamic balance of positive and negative regulators. It is known that VEGF-A, FGF, angiopoietins, transforming growth factor alpha and beta (TGF-α, TGF-β), hepatocyte growth factor (HGF), connective tissue growth factor (CTGF), interleukin-8 (IL-8) and LL-37 positively regulate angiogenesis whereas thrombospondin, angiostatin, endostatin, and pigment epithelium-derived factor (PEDF) negatively regulate angiogenesis. (Witmer, A.N., et al., Progress in Retinal and Eye Research, 22:1-29 (2003); Koczulla, R., et al., J. Clin. Invest., 111:1665-1672 (2001).) It is possible that angiogenic factors that have been shown to activate signaling pathways that are not truly independent of VEGF, may converge somewhere downstream, thus potentially bypassing an upstream inhibition of VEGF. Diversity is also described at the level of distinct roles for endothelial cells from different tissues. (Conway. E.M. and P. Carmeliet, Genome Biology, 5:207 (2004).) This suggests that an anti-angiogenic drug developed for one therapeutic indication, such as cancer, may not show the same efficacy in angiogenic mediated eye disease. It is clear that it will take years of research to unravel all of these interactions one by one. In contrast, the unbiased approach we are proposing here appears particularly well suited to identifying molecules that would block multiple and/or critical parts of these pathways.

VEGF at the cellular level mediates multiple functions, including survival, proliferation, migration, vascular permeability, tubulogenesis, NO and prostanoid synthesis, and gene expression. (Zachery, I., Biochem. Soc. Trans., 31:1171-1177 (2003); Cross, M.J., et al., Tr. Biochem. Sci., 28:488-494 (2003).) There is a growing body of knowledge that describes the key molecular players and the inter connectivity of cellular pathways governing the biology of angiogenesis. VEGF initiates a diverse, complex and integrated network of signaling pathways via its major receptor, KDR. (Carmelier, P., Nat. Med., 6:389-395 (2000); Ferrara, N., Curr. Opin. Biotechnol., 11:617-624 (2000).) The schematic (Figure 1) provides a map of our current understanding of KDR mediated signaling and the interplay between other membrane bound molecules and their downstream effectors. Many of these pathway nodes have been studied in isolation or sequentially to determine which key pathways are involved in angiogenesis. Upon ligand binding, KDR dimerizes and auto phosphorylates tyrosine residues in the cytoplasmic domain of the receptor. It is known that six tyrosines are phosphorylated but the function of many of these sites have not been defined. (Wu, L., et al., J. Biol. Chem., 275:6059-6062 (2000); Takahashi, T., et al., EMBO J., 20:2768-2778 (2001); He, H., et al., J. Biol. Chem., 274:25130-25135 (1999).) The phospho-tyrosine residues are postulated to be interaction sites of SH2 containing proteins, such as Grb2, Nck, Shc, SHP-1, SHP-2 and HCPTPA. The SH2 containing protein VEGFR-associated protein (VRAP) interacts with VEGF activated KDR via Tyr 951 and PLCγ binds at Tyr -1175. (Dougher-Vermazen, et al., Biochem. Biophys. Res. Commun., 205:728-738 (1994); Wu, L., et al., J. Biol. Chem., 275:6059-6062 (2000).)

A possible role for VRAP as an adaptor protein for shuttling PLCγ and PI₃K to KDR is proposed. It is also reported that c-Src may mediate PLCγ activation in VEGF dependent manner. (He, H., et al., J. Biol. Chem., 274:25130-25135 (1999).) It is well described in the literature that VEGF via KDR leads to activation of phospholipase C-γ (PLC γ), protein kinase C (PKC), Ca2+, ERK (extracellular-signal-regulated protein kinase), Akt, Src, focal adhesion kinase (FAK) and p38 MAPK pathways. (Takahashi, T., et al., EMBO J., 20:2768-2778 (2001); Zachary, 1. And G. Gliki, Cardiovasc. Res., 49:568-581 (2001); Gerber, H.P., et al., J. Biol. Chem., 273:30336-30343 (1998); Thakker, G.D., et al., J. Biol. Chem., 274:10002-10007 (1999); Abedi, H. and I, Zachary, J. Biol. chem., 272:15442-15451 (1997); Takahashi, T., et al., Oncogene, 18:2221-2230 (1999); Gliki, G., et al., Biochem. J., 353:503-512 (2001).) This leads to long term effects on gene expression, cell survival and migration. The growing body of data paints a very complicated picture of pathway networks.

Recently Koczulla *et al.,* reported a role for LL-37, a human cathelicidin antimicrobial peptide, and the G-protein coupled receptor FPRL-1 in angiogenesis. (Koczulla, R., et al., J. Clin. Invest., 111:1665-1672 (2003).) This pathway has been demonstrated to independently mediate angiogenesis. The FPRL-1 receptor is expressed on endothelial cells and LL-37 specifically mediates endothelial cell proliferation. (Koczulla, R., *et al.* (2003); Keitoko, M., et al., J. Mol. Cell Cardiol., 29:881-894 (1997).) Gene disruption of the mouse homolog, CRAMP, showed decreased vascularization during wound repair. (Koczulla, R. *et al.* (2003).) LL-37 has also been shown to function through the FPRL-1 receptor to chemoattract human peripheral blood neutrophils, monocytes and T cells. (Yang, D., et al., J. Exp. Med., 192:1069 (2000).) At this point the signaling mechanism is poorly understood, but there is evidence to suggest that LL-37 activates ERK1/2, p38 MAPK and JNK. (Koczulla, R., *et al.* (2003); Prenzel, N., et al., Nature, 402:884 (1999); Ferguson, S.S.G., et al., Science, 274:363-366 (1996).)

A role for heterotrimeric G proteins in the signaling pathways for Flt-1 and KDR has been reported in the literature. (Zeng, H., et al., J. Biol. Chem., 278:20738-20745 (2003); Zeng, H., et al., J. Biol. Chem., 277:46791-46798 (2002); Zeng, H., et al., J. Biol. Chem.; 277:4003-4009 (2002).) There is evidence that that the Gq/11 protein binds to the intracellular domain of KDR and up regulates its activity. (Zeng, H., *et al.* (2003).) It was observed that a Gq/11-specific antisense oligonucleotide blocks KDR phosphorylation, MAPK activation and cell proliferation. Other members of this superfamily including insulin receptor, insulin-like growth factor receptors, and PDGF receptor (Alderton, F., et al., J. Biol. Chem., 276:28578-28585 (2001); Kuemmerle, J.F. and K.S. Murthy, J. Biol. Chem., 276:7187-7194 (2001)) have been shown to utilize heterotrimeric G proteins for their signaling. These results hint at a larger, more integrated network for angiogenesis.

In the following examples, PAM was used to screen compounds to identify inhibitors of angiogenesis. For this purpose, eight assays were used in PAM: 1) KDR/VRAP or KDR/Gaq interaction cell based biosensor; 2) FPRL-1/β2-arrestin interaction cell based biosensor; 3) src kinase; 4) p38 MAPK; 5) Ca2+ flux or cAMP production; 6) Akt; 7) ERK1/2 cascade; and 8) phenotype (measure cell proliferation/cell survival). The following examples discuss the assays used and the methods of preparing cell lines for conducting these assays.

### Example 1: Preparation of Cell Based Biosensors

Construction of the cell based biosensor utilizes a system that takes advantage of complementation of two inactive deletion mutants of β-galactosidase, Δα and Δω (Mohler, W.A. and H.M. Blau, Proc. Natl. Acad. Sci. USA, 93:12423-12427 (1996)). These two complementing enzyme fragments have a low affinity for one another and are each enzymatically inactive. The two interacting proteins of interest, A and B, are expressed as fusion proteins to the two mutant forms of β-galactosidase. When A and B interact, the β-galactosidase mutants complement one another and form an active enzyme. If proteins do not interact, or are prevented from interacting, the β-galactosidase mutants remain inactive. The cell based biosensor provides a direct readout for the protein-protein interaction of interest. Unlike the yeast two hybrid method, the interaction is monitored directly in real time and within the physiologically relevant cellular compartment. Using this system, protein-protein interactions can be monitored by a simple β-galactosidase enzymatic assay using either a fluorescent or chemiluminescent readout (Rossi, F., et al., Proc. Natl. Acad. Sci. USA, 9r:8405-8410 (1997); Blakely, B.T., et al., Nature Biotech., 18:218-222 (2000); Rossi, F.M.V., et al., Methods Enzymol., 328:231-251 (2000)).

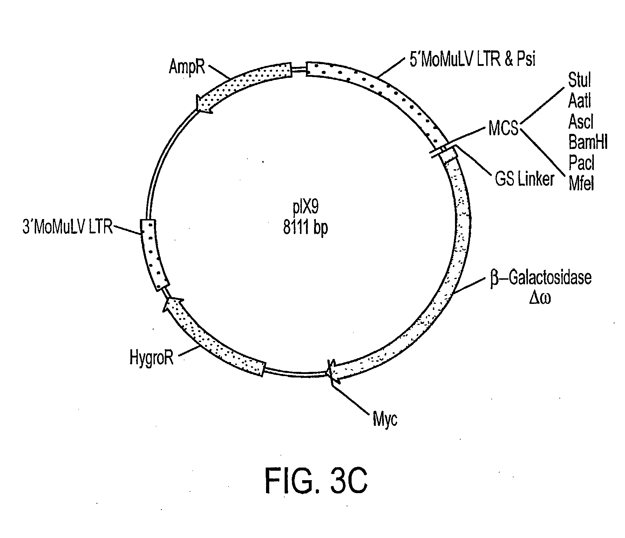

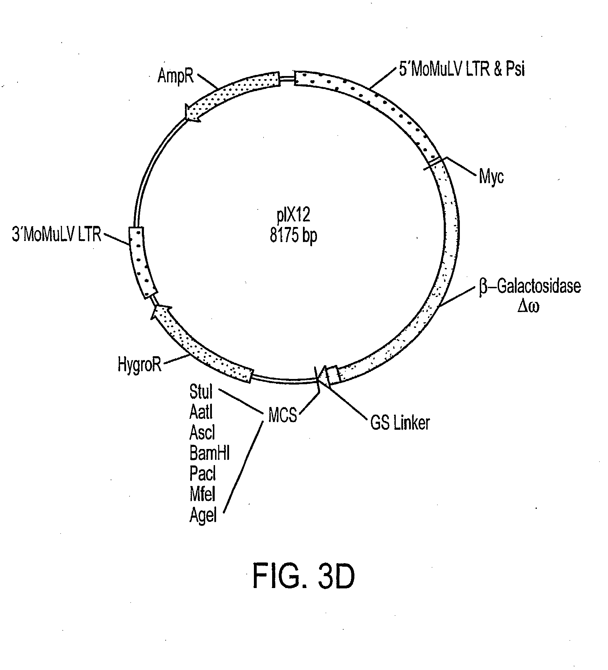

The generic protocol followed to construct the cell line is described here and in greater detail in Yan, Y-X., et al. (J. Biomol. Screen., 7:451-459(2002)) and Palmer, M., et al. (U.S. Patent No. 6,893,827). The InteraX™ vectors (see Figures 3A-3D) are commercially available from Applied Biosystems. To build a functional, cell-based assay for a given protein-protein interaction, the open reading frame of the first protein of interest is cloned into either the pIX3 or pIX6 vector (see Figures 3A and 3B, respectively) to allow for expression of that protein as a fusion protein with the Δα mutant of β-galactosidase. This will become the parental cell line into which multiple interaction fusion partners will be delivered by retroviral infection. The second protein of interest is cloned into either the pIX9 or pIX12 vector (see Figures 3C and 3D, respectively) to allow for expression of the second protein as a fusion protein with β-galactosidase Δω. To stream line assay construction the delta omega fusion partners may be generated in parallel starting with the KDR delta alpha parent. This has been implemented successfully for GPCR beta 2 arrestin interacting pairs using an arrestin parent cell line (unpublished, M. Palmer). Detection of beta gal activity uses a simple mix and read protocol that can be stopped at a certain time point and read up to 2 hours later by glow luminescence.

The technology for measurement of functional EGF receptor dimerization (Blakely, B.T., et al., Nature Biotech., 18:218-222 (2000); Graham, D.L., et al., J. Biomol. Screen., 6:401-411 (2001)), EGFr Grb2 interaction (unpublished results, M.Palmer) and beta 2 adrenergic receptor beta 2 arrestin interaction (Yan, Y-X., *et al.* (2002)) have been successfully implemented. A retroviral vector is used for delivery of the gene of interest but plasmid based vectors may also be used. It is important that the fusion proteins are not highly over-expressed as this will lead to constitutive interaction of the delta alpha and delta omega mutants. Expression below 10⁶ copies per cell is ideal. Retroviral expression tends to give lower expressing cell lines due to single site of integration into the cells DNA. The full length KDR receptor has been expressed as a fusion to both delta alpha and delta omega (unpublished results, M.Palmer). It is not known if problems with expression of FPRL-1, VRAP or Gαq will be encountered. It has been found by the inventors that sometimes fusion to delta alpha improves the expression of problematic proteins. The background cell of choice will be HUVEC cells, but alternative cell lines such as HMVEC (Cambrex) may also be utilized (Ades, E.W., et al., J. Invest. Dermatol., 99:683-690 (1992); Shao, R. and X. Guo, Biochem. Biophys. Res. Commun., 321:788-794 (2004)). HUVECs express both KDR and FPRL-1 endogenously. This has not interfered with biosensor function in the past (unpublished results). Transfection of these cells has become more successful through the use of new transfection reagents such as jetPEI-HUVEC (PolyPLus Transfection).

### Example 2: FPRL-1/β2-arrestin interaction biosensor

Most GPCRs are down-regulated upon ligand binding by the phosphorylation dependent interaction of beta-arrestin with the C-terminal cytosolic region of the receptor (Ferguson, S.S.G., et al., Science, 274:363-366 (1996); Krupnick, J.G., et al., J. Biol. chem., 272:18125-18131 (1997); Lohse, M.M., et al., Science, 248:1547-1550 (1990); Lohse, M.M., et al., J. Biol. Chem., 267:85558-85564 (1992); U.S. Patent No. 6,893,827). The receptor is targeted to clatherin coated pits for internalization. This common mechanism can be exploited to measure GPCR activation. FPRL-1 has been reported to be internalized in ligand dependent manner and the FPR receptor family has been shown to interact with arrestin. A generic method to measure GPCR arrestin interaction has been developed (Graham, D.L., et al., J. Biomol. Screen., 6:401-411 (2001); U.S. Patent 6,893,827). This direct measurement of receptor activation overcomes limitations of indirect methods such Ca 2+ flux or reporter gene readout. Indirect methods can suffer from false positives due to alternative GPCR receptor activation affecting the same pathway or cross talk between pathways. Cell line construction follows the previously published protocol as described in Yan et al. (Graham, D.L., et al., J. Biomol. Screen., 6:401-411 (2001); U.S. Patent 6,893,827).

### Example 3: KDR/VRAP interaction biosensor

VRAP is a 389-amino acid protein that contains a SH2 domain and a C-terminal Proline rich domain (Wu, L., et al., J. biol. Chem., 275:6059-6062 (2000)). It is expressed in human umbilical vein endothelial cells as well as numerous other tissues and cell types. It binds to KDR intracellular domain in a VEGF dependent manner. This interaction is poorly characterized and merits further study due to broad tissue expression pattern and potential role in KDR signaling. Expression of VRAP in recombinant systems has not been reported so this may present some challenges. Expression of proteins of similar size as fusions to the biosensor pairs has not been a problem in the past but it may be necessary to use a truncated version containing the SH2 domain. Cell line construction will follow the same protocol as described above.

### Example 4: KDR/Gαq interaction biosensor

A novel signaling pathway has been identified, which upon VEGF binding, KDR intracellular (IC) domain interacts with and activates a Gq/11 protein (Zeng, H., et al., J. Biol. Chem., 278:20738-20745 (2003)). It is proposed that the interaction of Gq/11 protein with KDR IC domain occurs upstream of receptor phosphorylation and that the release of the Gαq subunit upon activation of Gαq acts synergistically with KDR receptor phosphorylation to activate PLCγ and increased Ca2+ mobilization. This pathway could uncover a number of interesting new targets in angiogenesis. This biosensor would be constructed in a similar way to those previously described by infecting the KDR Δα or Δω mutant fusion parent cell line with a construct containing Gαq subunit fused to the second interacting mutant.

### Example 5: Testing and validation of the biosensor cell lines

Clones are selected based on fusion protein expression levels and the signal to background upon ligand induction. Expression levels of the fusion proteins can be estimated by Western analysis and quantified in a beta-galactosidase ELISA. The clones are tested for an increase in beta gal activity upon stimulation with VEGF or LL-37. Dose response of the *KDR*/*VRAP* and *KDR*/*Gαq* cell lines to VEGF-A (121 and 165) (1-20nM), other growth factors (VEGF-B, EGF, PIGF) and neutralizing antibody (MAB3572, R & D Sytems) are tested to determine specificity of response. Similarly FPRL-1/β2-arrestin will be tested for dose response to LL-37 (1-20ug/ml), other known ligands such the W peptide (WKYMVm) (0.5-20 ug/ml) and antiserum against FPRL-1 (Koczulla, R., et al., J. Clin. Invest., 111:1665-1672 (2001)).

### Example 6: Kinase Assays (Src, p38MAPK, Akt, ERK)

A number of kinases play key roles in the downstream signaling of KDR. The mitogenic pathway is strongly influenced by PKC and ERK. cSrc is implicated in a number of pathways due to its reported effects on PLCγ and FAK activity (He, H., et al., J. Biol. Chem., 274:25130-25135 (1999);Abu-Ghazaleh, R., et al., Biochem. J., 360:255-266 (2001)). Akt and p38MAPK play key roles in the VEGF mediated long term survival and migration of endothelial cells (Gerber, H.P., et al., J. biol. Chem., 273:30336-30343 (1998); Thakker, G.D., et al., J. Biol. Chem., 274:10002-10007 (1999)). There are also a number of well characterized inhibitors against these kinases to act as probes in validation of this pathway assay matrix. The method chosen for detection of changes in kinase activity will need to be sensitive enough to measure endogenous levels of activity. Based on preliminary results the sensitivity requires that ELISA based methods be used. There is a large library of Phospho specific antibodies (Cell Signaling and Biosource) that can selectively capture and quantify by ELISA the endogenous phosphate incorporation of a specific kinase. Each ELISA is optimized to produce uniformity in protocol, cell number, sensitivity and readout time. Most commercial kits utilize colormetric or fluorescent substrate for the detection conjugate. Modification to use a chemiluminescent substrate will improve sensitivity and robustness. Cell Signaling and Biosource provide commercially available kits for Src, p38 MAPK, Akt and ERK that can be quickly profiled for best performance in the matrix.

### Example 7: Ca2+ flux or cAMP production

FPRL1 belongs to the seven transmembrane domain Gi-protein-coupled receptor family. The stimulation of FPRL-1 with LL-37 on HUVECs has been shown to be pertussis toxin-sensitive (Koczulla, R., et al., J. clin. Invest., 111:1665-1672 (2003)), indicating coupling to one or more members of the Gi (inhibitory G) subfamily of G proteins. Activation of PLCγ leads to generation of diacylglycerol (DAG) and inositol 1,4,5-trisphosphate (IP3). Calcium is released from intracellular stores in response to PLCγ activation, influencing downstream production of prostacyclin and NO (Gliki, G., et al., Biochem. J., 353:503-512 (2001)). Standard methods for fluorescent measurement of intracellular Ca2+ levels will be applied using dyes such as Fura-2, Fluo-4 and Calcium-Green. The cells are preloaded with dye and then time points are taken to determine base line and time window for measurement of calcium flux. Response is rapid and maximal response is obtained in less than one minute. Activation of FPRL-1 also down regulates cAMP production. An alternative second messenger assay would quantify changes in the level of cAMP produced from the conversion. In order to measure inhibiton of cAMP the cells must first be primed with forskolin to stimulate cAMP. This is an ELISA based method which could be adapted to chemiluminescent readout. (Applied Biosystems, DiscoverX).

### Example 8: Phenotype Assay

The cellular phenotypes, cell proliferation and/or cell death are measured using viable cell number determination methods. Measurement of cell proliferation and cytotoxicity using the luminescent assay ViaLight™-HS High Sensitivity Cytotoxicity and Cell Proliferation BioAssay Kit from Cambrex (East Rutherford, NJ) is optimized for HUVECs. Linear detection of cell proliferation or cell survival is possible from as few as 100 and up to 10⁶ cells. The typical signal to noise seen for HUVEC proliferation over 48 hrs at 20nM VEGF is 2-fold with a standard deviation of 10%. An alternative measurement of both ATP and ADP levels provides a ratio measurement indicating cells proliferation versus cell death.

### Example 9: The Pathway Assay Matrix (PAM)

The eight assays described above will be incorporated into a 96-well microplate assay matrix. Columns 1-8 in Figure 4A represent the individual assay nodes (alternatively, if more assay nodes are to be tested, the 96-well microplate can be used such that up to 12 assay nodes are tested). HUVEC cells would be grown in 48 well plates to a subconfluent optimal density (100,000 cells). Cell treatment and compound testing occurs in these wells. At given time endpoints cell lysates will be transferred for individual assay readout to the 96 well PAM assay system. The optimization will involve testing of endpoint times, biosensor cross comparisons, correlation with other cell based readouts of the pathways such as proliferation and migration assays. Reagent addition will also require optimization to simplify the assay workflow. A robot platform could be programmed to handle all reagents additions in a timed fashion. Optimal assay endpoints will be explored to cover a timeline from 1 minute to 24 hours in reasonable increments. Both the specificity and the potency on downstream signaling will be important measurements. It is assumed at this point that time points will be selected where data shows the greatest differential for 3 node endpoints. It may also be the case that a given node does not provide a sufficiently differentiating response over time or in response to various factors. It may require that a new node replace it. This may or may not fit into the required timelines and we would proceed with only 7 nodes. Insufficient overage of the key angiogenesis signaling pathways with less than 7 nodes would occur. It is important to measure the pathway proximal to the membrane and then farther downstream to maximize node response and internal controls. For example inhibiting KDR/VRAP interaction would have downstream effects on PLC, Ca mobilization, PI3K and AKT activity. Inhibiting PI3K will exhibit changes in AKT activity and cell viability.

The data analysis and visualization software, Spotfire™, allows one to efficiently upload results from Excel and effectively analyze the data to cluster small molecule inhibitor activity based on both expected activities but also uncover unexpected relationships. It has already been applied to pathway clustering analysis of gene expression array data and small molecule structure activity analysis for lead optimization (Shapshak, P., et al., Front Biosci., 9:2935-2946 (2004); Cunningham, M.J., J. Pharmacol. Toxicol. Methods, 44:291-300 (2000)). Analysis at the level of multiple pathway nodes and time points to gain greater understanding of pathway dynamics has been described for EGFr signaling in the literature (Kholodenko, B.N., et al., J. Biol. Chem., 274:30169-30181 (1999)).

### Example 10: Testing of angiogenesis inhibitors

Eight commercially available compounds were selected to act as molecular probes of the target nodes. These molecules have been reported to have an inhibitory effect on relevant target nodes selected in the KDR and FPRL-1 pathway. In addition, a 284 compound GPCR directed library will be tested in PAM in a dose dependent and time dependent manner. The calibrating compounds in Table 1 are selected based on the following criteria: (1) activity is reported in the literature; (2) exhibits cell permeable characteristics; (3) exhibits > 5 fold selectivity for reported target and; (4) the target is a characterized node in the KDR or FPRL-1/LL37 signaling pathway. These compounds are not meant to act as drug leads but rather to provide validation of the assay platform based on reported results and provide a data set on which to begin development of the data analysis tools. The compounds CNRX0167, SU5416, Go6976, SB 203580 have been tested *in vivo* and shown activity against the relevant target and pathway (Gelaw, B. and S. Levin, Surgery, 130:497-501 (2001); Patel, N., et al., J. Pharmacol. Exp. Ther., 306:838-845 (2003); Martiny-Baron, G., et al., J. Biol. Chem., 268:9194-9197 (1993); Jacob, T., et al., Eur. J. Vasc. Endovasc. Surg., 29:470-478 (2005)). SU5416 has been shown to block angiogenesis in the wound-healing model (Gelaw, B., *et al.* (2001)). The compound fingerprint of interesting compounds should show efficacy in blocking both cell proliferation and permeability, the two main indicators of progression in neovascularization. The off target effects will also be important at higher concentrations in order to assess the pattern generated for both selective and non-selective classes of active compounds. Compounds will be tested at and above the reported IC₅₀ or Ki.

**Table 1-Inhibitor list**

| **Compound** | **Target** | **Reported Potency** | **Selectivity** |
|---|---|---|---|
| CNRX0167 | VEGFR-2 kinase inhib | 70nM | Does not inhibit PDGF, EGF, and IGF-1 RTK >100uM |
| SU5416 | VEGFR-2 and PDGF-R kinase inhib | IC50 = 1.04uM and 20 uM | ATP-competitive inhibitor of VEGF-R (KDR/Flk-1) and PDGF-R tyrosine kinases |
| SH-6 or SH-5 | AKT inhibitor | | Does not decrease phosphorylation of PDK-1 or other kinases downstream of Ras, such as MAPK |
| Go 6976 | PKC □ and PKC □1 inhibitor | PKC □ (IC50 = 2.3 nM) PKC□I (IC50 = 6.2 nM) | > uM no effect on the kinase activity of PKC subtypes □, □ and □. |
| SL327 | MEK 1/2 inhibitor | MEK 1/2 (IC50 = 180 nM and 220 nM) | inhibit AP-1 (IC50 = 2.03 uM) and ERK1, MKK3/p38, MKK4, JNK, and PKC activities at much higher concentrations (IC50 > 10 uM |
| SB 203580 | p38 MAP kinase inhibitor | 600 nM in cells | Does not inhibit the MAP kinase homologs JNK and p42 MAP kinase |
| CNRX0054 | Raf1 Kinase Inhibitor | (IC50 = 9 nM) | Shows ≥100-fold selectivity versus Cdk1, Cdk2, c-Src, ERK2, MEK, p38, Tie2, VEGFR2, and c-Fms |
| Wortmannin | PI3K inibitor | IC50 = 5 nM | 100-fold higher inhibits other kinases |

The number of compounds tested does not require a fully automated protocol utilizing high throughput robotics. A 96-well microplate pipeting station is utilized to synchronize assay start and stop times as well as the addition of compounds and reagents to the assay wells.

### Example 11: Testing of active biomolecules in an in vivo model

Two to three active biomolecules found in Example 10 are then tested in the wound angiogenesis assay, to determine correlation of effects seen in the PAM system. *This in vivo* model provides a quantification of the reduction in capillary growth achieved by the selected compounds. The wound model is used to assess the potential of these molecules to inhibit spontaneous angiogenesis that is a multifactorial process not solely dependent on VEGF. Although the dynamics and regulation of the cellular biology of wound healing may differ somewhat from the underlying cause of neovascularization in the retina, is well understood that VEGF signaling mediated through the VEGFR-2 /KDR receptor is clearly implicated in the progression of diabetic macular edema and in "wet" AMD.

## Claims

1. A method of screening to identify inhibitors of angiogenesis comprising:
- treating primary cells or cell lines, with the proviso that the primary cells or cell lines are not human embryonic stem cells or cell lines, in tissue culture plate wells or vessels with a drug of interest selected from a small molecule, a siRNA, a protein, a peptide, an antibody, an antibody fragment, or an aptamer, wherein at least two different primary cells or cell lines are treated, each in their own tissue culture plate well or vessel;
- lysing the primary cells or cell lines;
- adding the cell lysates to eight or more wells of a microarray plate, with the caveat that cell lysate from only one primary cell or cell line is present in any single well;
- adding eight or more reagents to the wells of said microarray plate occupied by a primary cell, cell line or cell lysate, wherein at least one reagent is added to each of the wells of said microarray plate occupied by a cell lysate;
- conducting an assay reaction for each well of said microarray plate, wherein each reagent and or assay is chosen to measure a particular node in the disease pathway for the disease for which drugs are being screened, wherein at least eight assays are performed, and wherein said assays are 1) a KDR/VRAP or KDR/Gαq interaction cell based biosensor assay; 2) a FPRL- 1/β2-arrestin interaction cell based biosensor assay; 3) a src kinase assay; 4) p38 MAPK assay; 5) Ca2+ flux or cAMP production assay; 6) an Akt assay; 7) an ERK1/2 cascade assay; and 8) an assay of phenotype (measure cell proliferation/cell survival); and
- analysing whether the drug of interest inhibits each of the nodes of the disease pathway, thereby determining whether the drug of interest would be effective as an inhibitor of angiogenesis.

2. The method of claim 1, wherein the dose of said drug of interest varies from cell culture to cell culture.

3. The method of claim 1, wherein at least one well in each lane of the microarray plate contains a cell lysate from an untreated primary cell or cell line and one or more reagents.

4. A method according to claim 1 wherein the step of
- adding eight or more reagents to eight or more wells of a microarray plate, wherein at least one reagent is added to each of the wells of said microarray plate occupied by a cell lysate; takes place before the step of:
- adding the cell lysates to the eight or more wells of a microarray plate containing a reagent, with the caveat that cell lysate from only one primary cell or cell line is present in any single well.

5. The method of claim 4, wherein there are eight or more wells of the microarray plate that contain lysate from an untreated primary cell or cell line together with one or more reagents, and each of the assays performed on the lysates from treated primary cells or cell lines are also performed on the lysates from untreated primary cells or, cell lines as a control.

6. A method according to claim 1 wherein treating the primary cells or cell lines takes place in eight or more tissue culture plate wells or vessels, wherein at least two different primary cells or cell lines are treated, each in their own tissue culture plate well or vessel; and following lysing of the primary cells or cell lines:
- the eight or more reagents are added to the eight or more tissue culture plate wells or vessels, wherein at least one reagent is added to each of the tissue culture plate wells or vessels; and subsequently
- the reagents and the treated lysates are added to the eight or more wells of a microarray plate, with the caveat that lysate from only one primary cell or cell line is present in any single well.

7. The method of claim 6, wherein there are eight or more wells of the microarray plate that contain lysate from an untreated primary cell or cell line together with one or more reagents, and each of the assays performed on the lysate from treated primary cells or cell lines are also performed on the lysates from untreated primary cells or cell lines as a control.

8. A method according to claim 1 wherein the step of conducting an assay reaction takes place in each well or vessel of said tissue culture plate.

9. The method of claim 8 wherein there are eight or more wells or vessels of the tissue culture plate that contain lysate from an untreated primary cell or cell line together with one or more reagents, and each of the assays performed on the lysates from treated primary cells or cell lines are also performed on the lysates from untreated primary cells or cell lines as a control.

10. The method of claim 1, 4, 6 or 8, wherein said cell lysate is selected from the group consisting of cell lysate obtained from a culture of HMVEC cells and cell lysate obtained from a culture of HUVEC cells.

11. The method of claim 1, 4, 6 or 8, wherein said reagent is selected from the group consisting of essential and nonessential amino acids, inorganic salts, organic compounds, trace elements, serum, growth factors, antibiotics, and vitamins.

## Patentansprüche

1. Screening-Verfahren zur Identifizierung von Angiogenese-Inhibitoren, welches umfasst:
- Behandeln primärer Zellen oder Zelllinien, mit der Maßgabe, dass die primären Zellen oder Zelllinien keine humanen embryonalen Stammzellen oder Zelllinien sind, in Gewebekulturschalen-Wells oder -Gefäßen mit einem Wirkstoff von Interesse, ausgewählt aus einem kleinen Molekül, einer siRNA, einem Protein, einem Peptid, einem Antikörper, einem Antikörperfragment oder einem Aptamer, wobei mindestens zwei unterschiedliche primäre Zellen oder Zelllinien behandelt werden, jeweils in ihrem eigenen Gewebekulturschalen-Well oder -Gefäß,
- Lysieren der primären Zellen oder Zelllinien,
- Hinzufügen der Zelllysate in acht oder mehr Wells einer Mikroarray-Schale, mit dem Vorbehalt, dass nur Zelllysat aus einer primären Zelle oder Zelllinie in jedem einzelnen Well vorhanden ist,
- Hinzufügen von acht oder mehr Reagenzien in die Wells der Mikroarray-Schale, welche durch eine primäre Zelle, Zelllinie oder ein Zelllysat belegt sind, wobei mindestens ein Reagens in jedes der Wells der Mikroarray-Schale, welche durch ein Zelllysat belegt sind, hinzugefügt wird,
- Durchführen einer Assay-Reaktion für jedes Well der Mikroarray-Schale, wobei jedes Reagens oder jeder Assay ausgewählt wird, um einen bestimmten Knotenpunkt in dem Krankheits-Weg der Krankheit, für welche die Wirkstoffe gescreent werden, zu messen, wobei mindestens acht Assays durchgeführt werden, und wobei die Assays sind: 1) ein Zell-basierter KDR/VRAP- oder KDR/Gαq-Interaktions-Biosensor-Assay, 2) ein Zell-basierter FPRL-1/β2-Arrestin-Interaktions-Biosensor-Assay, 3) ein src-Kinase-Assay, 4) p38-MAPK-Assay, 5) Ca2+-Fluss- oder cAMP-Produktions-Assay, 6) ein Akt-Assay, 7) ein ERK1/2-Kaskade-Assay und 8) ein Phänotyp-Assay (Messen von Zellproliferation/Zellüberleben), und
- Analysieren, ob der Wirkstoff von Interesse jeden der Knotenpunkte des Krankheits-Wegs inhibiert, und dadurch Bestimmen, ob der Wirkstoff von Interesse als Angiogenese-Inhibitor wirksam wäre.

2. Verfahren gemäß Anspruch 1, wobei die Dosis des Wirkstoffs von Interesse von Zellkultur zu Zellkultur variiert.

3. Verfahren gemäß Anspruch 1, wobei mindestens ein Well in jeder Reihe der Mikroarray-Schale ein Zelllysat aus einer unbehandelten primären Zelle oder Zelllinie und ein oder mehrere Reagenzien enthält.

4. Verfahren gemäß Anspruch 1, wobei der Schritt von
- Hinzufügen von acht oder mehr Reagenzien zu acht oder mehr Wells einer Mikroarray-Schale, wobei mindestens ein Reagens zu jedem der Wells der Mikroarray-Schale, die durch ein Zelllysat belegt sind, vor dem Schritt von:
- Hinzufügen der Zelllysate in acht oder mehr Wells einer Mikroarray-Schale, welche ein Reagens enthalten, mit dem Vorbehalt, dass nur Zelllysat aus einer primären Zelle oder Zelllinie in jedem einzelnen Well vorliegt, stattfindet.

5. Verfahren gemäß Anspruch 4, wobei acht oder mehr Wells der Mikroarray-Schale vorhanden sind, welche Lysat aus einer unbehandelten primären Zelle oder Zelllinie gemeinsam mit einem oder mehreren Reagenzien enthalten, und jeder der Assays, welcher auf die Lysate von behandelten primären Zellen oder Zelllinien angewandt wird, ebenfalls auf die Lysate von unbehandelten primären Zellen oder Zelllinien als Kontrolle angewandt wird.

6. Verfahren gemäß Anspruch 1, wobei die Behandlung der primären Zellen oder Zelllinien in acht oder mehr Gewebekulturschalen-Wells oder -Gefäßen stattfindet, wobei mindestens zwei unterschiedliche primäre Zellen oder Zelllinien behandelt werden, jeweils in ihrem eigenen Gewebekulturschalen-Well oder -Gefäß, und wobei nach nachfolgend an die Lyse der primären Zellen oder Zelllinien die folgenden Schritte stattfinden:
- die acht oder mehr Reagenzien werden in die acht oder mehr Gewebekulturschalen-Wells oder -Gefäße hinzugefügt, wobei mindestens ein Reagens in jedes der Gewebekulturschalen-Wells oder -Gefäße hinzugefügt wird, und nachfolgend
- werden die Reagenzien und die behandelten Lysate in die acht oder mehr Wells einer Mikroarray-Schale hinzugefügt, unter dem Vorbehalt, dass nur Lysat aus einer primären Zelle oder Zelllinie in jedem einzelnen Well vorliegt.

7. Verfahren gemäß Anspruch 6, wobei acht oder mehr Wells der Mikroarray-Schale vorliegen, welche Lysate von einer unbehandelten primären Zelle oder Zelllinie gemeinsam mit einem oder mehreren Reagenzien enthalten, und wobei jeder der Assays, welcher auf das Lysat von behandelten primären Zellen oder Zelllinien angewandt wird, auch auf die Lysate von unbehandelten primären Zellen oder Zelllinien als Kontrolle angewandt wird.

8. Verfahren gemäß Anspruch 1, wobei der Schritt der Durchführung einer Assay-Reaktion in jedem Well oder Gefäß der Gewebekulturschale stattfindet.

9. Verfahren gemäß Anspruch 8, wobei acht oder mehr Wells oder Gefäße der Gewebekulturschale vorliegen, welche Lysat von einer unbehandelten primären Zelle oder Zelllinie gemeinsam mit einem oder mehreren Reagenzien enthalten, und wobei jeder der Assays, welcher auf die Lysate von behandelten primären Zellen oder Zelllinien angewandt wird, ebenfalls auf die Lysate von unbehandelten primären Zellen oder Zelllinien als Kontrolle angewandt wird.

10. Verfahren gemäß Anspruch 1, 4, 6 oder 8, wobei das Zelllysat ausgewählt wird aus der Gruppe, bestehend aus Zelllysat, welches aus einer Kultur von HMVEC-Zellen erhalten wird, und Zelllysat, welches aus einer Kultur von HUVEC-Zellen erhalten wird.

11. Verfahren gemäß Anspruch 1, 4, 6 oder 8, wobei das Reagens ausgewählt wird aus der Gruppe, bestehend aus essentiellen und nichtessentiellen Aminosäuren, anorganischen Salzen, organischen Verbindungen, Spurenelementen, Serum, Wachstumsfaktoren, Antibiotika und Vitaminen.

## Revendications

1. Procédé de criblage à la recherche d'inhibiteurs de l'angiogenèse, comprenant :
- le traitement de cellules primaires ou de lignées cellulaires primaires, étant entendu que les cellules ou lignées cellulaires primaires ne sont pas des cellules souches embryonnaires humaines ou des lignées de cellules souches embryonnaires humaines, dans des puits ou récipients de plaques pour culture de tissus, par une substance active d'intérêt choisie parmi une petite molécule, un ARNsi, une protéine, un peptide, un anticorps, un fragment d'anticorps, ou un aptamère, au moins deux cellules ou lignées cellulaires primaires différentes étant traitées, chacune dans son propre puits ou récipient de plaque pour culture de tissus ;
- la lyse des cellules ou lignées cellulaires primaires ;
- l'introduction des lysats cellulaires dans au moins huit puits d'une plaque à microréseaux, étant entendu que dans tout puits individuel est présent le lysat cellulaire ne provenant que d'une seule cellule ou lignée cellulaire primaire ;
- l'introduction d'au moins huit réactifs dans les puits de ladite plaque à microréseaux occupés par une cellule primaire, une lignée cellulaire primaire ou un lysat cellulaire, au moins un réactif étant introduit dans chacun des puits de ladite plaque à microréseaux occupés par un lysat cellulaire ;
- la conduite d'une réaction de dosage pour chaque puits de ladite plaque à microréseaux, chaque réactif et/ou chaque dosage étant choisi pour mesurer un noeud particulier dans une voie pathogénique pour la maladie pour laquelle des substances actives sont recherchées, au moins huit dosages étant effectués, et lesdits dosages étant 1) un dosage par biocapteurs basé sur des cellules à interaction KDR/VRAP ou KDR/Gaq ; 2) un dosage par biocapteurs basé sur des cellules à interaction FPRL-1/arrestine β2 ; 3) un dosage de src kinase ; 4) le dosage p38 MAPK ; 5) le dosage de flux Ca²⁺ ou la production d'AMPc ; 6) un dosage Akt ; 7) un dosage de cascade ERK1/2 ; et 8) un dosage de phénotype (dosage de prolifération cellulaire/survie de cellules) ; et
- une analyse visant à déterminer si la substance active d'intérêt inhibe chacun des noeuds de la voie pathogénique, permettant de déterminer si la substance active d'intérêt pourrait être efficace en tant qu'inhibiteur de l'angiogenèse.

2. Procédé selon la revendication 1, dans lequel la dose de ladite substance active varie d'une culture de cellules à l'autre.

3. Procédé selon la revendication 1, dans lequel au moins un puits dans chaque rangée de la plaque à microréseaux contient un lysat cellulaire provenant d'une cellule ou lignée cellulaire primaire non traitée et un ou plusieurs réactifs.

4. Procédé selon la revendication 1, dans lequel l'étape
- d'introduction d'au moins huit réactifs dans au moins huit puits d'une plaque à microréseaux, au moins un réactif étant introduit dans chacun des puits de ladite plaque à microréseaux occupés par un lysat cellulaire, a lieu avant l'étape
- d'introduction des lysats de cellules dans lesdits au moins huit puits d'une plaque à microréseaux contenant un réactif, étant entendu que dans tout puits individuel est présent le lysat cellulaire ne provenant que d'une seule cellule ou lignée cellulaire primaire.

5. Procédé selon la revendication 4, dans lequel il existe au moins huit puits de la plaque à microréseaux qui contiennent du lysat provenant d'une cellule ou lignée cellulaire primaire non traitée, conjointement avec un ou plusieurs réactifs, et chacun des dosages effectués sur les lysats provenant de cellules ou lignées cellulaires primaires traitées est également effectué sur les lysats provenant de cellules ou lignées cellulaires primaires non traitées, en tant que témoin.

6. Procédé selon la revendication 1, dans lequel le traitement des cellules ou lignées cellulaires primaires a lieu dans au moins huit puits ou récipients de plaque pour culture de tissus, au moins deux cellules ou lignées cellulaires primaires différentes étant traitées, chacune dans son propre puits ou récipient de plaque pour culture de tissus ; et après lyse des cellules ou lignées cellulaires primaires :
- on introduit lesdits au moins huit réactifs dans lesdits au moins huit puits ou récipients de plaque pour culture de tissus, au moins un réactif étant introduit dans chacun des puits ou récipients de plaque pour culture de tissus ; et ensuite
- on introduit les réactifs et les lysats traités dans lesdits au moins huit puits d'une plaque à microréseaux, étant entendu que dans tout puits individuel est présent le lysat ne provenant que d'une seule cellule ou lignée cellulaire primaire.

7. Procédé selon la revendication 6, dans lequel il existe au moins huit puits de la plaque à microréseaux qui contiennent du lysat provenant d'une cellule ou lignée cellulaire primaire non traitée, conjointement avec un ou plusieurs réactifs, et chacun des dosages effectués sur les lysats provenant de cellules ou lignées cellulaires primaires traitées est également effectué sur les lysats provenant de cellules ou lignées cellulaires primaires non traitées, en tant que témoin.

8. Procédé selon la revendication 1, dans lequel l'étape de conduite d'une réaction de dosage a lieu dans chaque puits ou récipient de ladite plaque pour culture de tissus.

9. Procédé selon la revendication 1, dans lequel il existe au moins huit puits ou récipients de la plaque pour culture de tissus qui contiennent du lysat provenant d'une cellule ou lignée cellulaire primaire non traitée, conjointement avec un ou plusieurs réactifs, et chacun des dosages effectués sur les lysats provenant de cellules ou lignées cellulaires primaires traitées est également effectué sur les lysats provenant de cellules ou lignées cellulaires primaires non traitées, en tant que témoin.

10. Procédé selon la revendication 1, 4, 6 ou 8, dans lequel ledit lysat cellulaire est choisi dans le groupe constitué par un lysat cellulaire obtenu à partir d'une culture de cellules HMVEC et un lysat cellulaire obtenu à partir d'une culture de cellules HUVEC.

11. Procédé selon la revendication 1, 4, 6 ou 8, dans lequel ledit réactif est choisi dans le groupe constitué par des acides aminés essentiels et des acides aminés non essentiels, des sels inorganiques, des composés organiques, des oligoéléments, le sérum, des facteurs de croissance, des antibiotiques, et des vitamines.
